# EUROPEAN PATENT APPLICATION

(11) **EP 4 053 849 A1**
(43) Date of publication of application: **07.09.2022**
(21) Application number: 20881377.4
(22) Date of filing: 30.03.2020
(51) Int. Cl.: G16H 20/30

(54) **EXERCISE ASSISTANCE DEVICE, EXERCISE ASSISTANCE SYSTEM, EXERCISE ASSISTANCE METHOD, AND PROGRAM**

(30) Priority: 01.11.2019 JP 2019199834
(71) Applicant: Astellas Pharma Inc., Chuo-ku Tokyo 103-8411 (JP)
(72) Inventor: KANAYAMA, Motohiro, Tokyo 103-8411 (JP)
(74) Representative: V.O.
(86) International application number: PCT/JP2020/014552
(87) International publication number: WO 2021/084779

(57) **Abstract**

An exercise support device (10) includes an acquisition unit (11A) that acquires, from a medical institution, confirmation result information indicating a confirmation result regarding execution of an exercise of a patient who has visited the medical institution at an exercise facility in which the exercise by an exercise program according to a disease of the patient is performed, and an execution unit (11B) that executes a process of transmitting, to both the exercise facility and the patient, permission or non-permission information of the execution of the exercise according to the exercise program of the patient at the exercise facility, on the basis of the confirmation result information acquired by the acquisition unit (11A).

## Description

### Technical Field

The technology of the present disclosure relates to an exercise support device, an exercise support system, an exercise support method, and a program.

### Background Art

Conventionally, as a technique that can be applied to a patient having a disease such as diabetes or metabolic syndrome to improve the disease, there have been the following techniques.

That is, Patent Document 1 discloses a user health management method for the purpose of providing customized health management so that a user can effectively manage health. The user health management method includes the steps of: receiving data including health-related information from a data source using short-range wireless communication; determining health-related behavior information of a user based on the health-related information; generating a health management recommendation for the user based on the health-related behavior information; and outputting the health management recommendation.

In addition, Patent Document 2 discloses a health management system aimed at enabling a user who needs to lose weight, such as a patient with metabolic syndrome, to continuously practice life improvement and an exercise program while enjoying it. The health management system includes: a service management server device including an RPG progress management function part configured to manage a roll playing game (RPG) content linked with various fitness devices, a use registration management function part configured to register a user, a schedule management function part configured to manage a schedule of a home appliance or the like, a food distribution management function part configured to manage a food distribution content, and an approval management function part configured to approve and manage a management content of the RPG progress management function part, and the food distribution management function part; an RPG content progress management terminal configured to display a management content of the RPG progress management function part; a food distribution management terminal configured to display a food distribution content managed by the food distribution management function part, and the like; a schedule management terminal configured to display a schedule content managed by the schedule management function part; and an approval management terminal configured to accept approval of a management content by the approval management function part.

Furthermore, Patent Document 3 discloses a health and consumption information management device intended to be able to transmit advice and messages for improving a lifestyle necessary for a user to live a healthy life and continuing exercise. The health and consumption information management device is a health and consumption information management device that provides advice and a message for living a healthy life from health and consumption information of a user, input from a terminal device. The health and consumption information management device includes a management means for managing health and consumption information input from the terminal device via a data communication network or by directly controlling the health and consumption information, and a control means for outputting advice on health and exercise for living a healthy life from the health information input to the management means, and transmitting a message according to each user from information obtained by integrating health and consumption.

### Citation List

### Patent Document

Patent Document 1: Japanese Patent Application Laid-Open (JP-A) No. 2013-218699
Patent Document 2: Japanese Patent Application Laid-Open (JP-A) No. 2009-140475
Patent Document 3: Japanese Patent Application Laid-Open (JP-A) No. 2002-297775 A

### SUMMARY OF INVENTION

### Technical Problem

A patient with diabetes, metabolic syndrome, or the like performs exercise at an exercise facility such as a sports club, a fitness club, or a sports gym using an exercise program corresponding to the condition of the patient's disease. In this case, it is necessary to reliably prevent a target patient from performing an exercise at an exercise facility that has not been confirmed by a medical institution as to whether or not the target patient can perform the exercise at the exercise facility in order to perform an effective exercise.

On the other hand, the techniques disclosed in the respective documents of Patent Documents 1 to 3 can contribute to the achievement of the object described in the corresponding document. However, in the techniques disclosed in each of Patent Document 1 to 3, for example, it is not always possible to reliably prevent the target patient from performing exercise at an exercise facility, which is not confirmed by a medical institution as to whether or not the target patient can perform exercise at the exercise facility.

The disclosed technique has been made in view of the above circumstances, and an object thereof is to provide an exercise support device, an exercise support system, an exercise support method, and a program capable of reliably preventing exercise that has not been confirmed by a medical institution as to whether or not a patient with diabetes, metabolic syndrome, or the like can perform exercise at an exercise facility.

### Solution to Problem

A first aspect of the present disclosure is an exercise support device including: an acquisition unit that acquires, from a medical institution, confirmation result information indicating a confirmation result regarding execution of an exercise of a patient who has visited a medical institution at an exercise facility in which the exercise is performed by an exercise program according to a disease of the patient; and an execution unit that executes a process of transmitting, to at least one of the exercise facility or the patient, permission or non-permission information of the execution of the exercise according to the exercise program of the patient at the exercise facility based on the confirmation result information acquired by the acquisition unit.

A second aspect of the present disclosure is an exercise support system including: the exercise support device according to the first aspect; an information reception device provided in the medical institution and including a reception unit that receives the confirmation result information and a transmission unit that transmits the confirmation result information to the exercise support device; and an information processing device provided at the exercise facility and including a processing unit that performs processing according to a process of transmitting permission or non-permission information for execution of an exercise according to the exercise program at the exercise facility, by the execution unit in the exercise support device.

A third aspect of the present disclosure is an exercise support method including: acquiring, from the medical institution, confirmation result information indicating a confirmation result regarding execution of an exercise of a patient who has visited the medical institution at an exercise facility in which the exercise according to an exercise program based on a disease of the patient is performed; and executing a process of transmitting, to at least one of the exercise facility or the patient, permission or non-permission information of the execution of the exercise according to the exercise program of the patient at the exercise facility based on the acquired confirmation result information.

A fourth aspect of the present disclosure is a program for causing a computer to execute a process of: acquiring, from a medical institution, confirmation result information indicating a confirmation result regarding execution of an exercise performed by a patient who has visited the medical institution, at the exercise facility in which the exercise according to an exercise program based on a disease of the patient is performed; and transmitting, to at least one of the exercise facility or the patient, permission or non-permission information of the execution of the exercise according to the exercise program of the patient at the exercise facility based on the acquired confirmation result information.

### Advantageous Effects of Invention

As described above, according to the technique of the present disclosure, it is possible to reliably prevent a patient from performing an exercise at an exercise facility that has not been confirmed by a medical institution as to whether or not the patient can perform the exercise at the exercise facility.

### BRIEF DESCRIPTION OF DRAWINGS

Fig. 1 is a block diagram illustrating an example of a hardware configuration of an exercise support system according to an embodiment.
Fig. 2 is a block diagram illustrating an example of a functional configuration of the exercise support system according to the embodiment.
Fig. 3 is a schematic diagram illustrating an example of a configuration of an exercise program database according to the embodiment.
Fig. 4 is a schematic diagram illustrating an example of a configuration of a patient information database according to the embodiment.
Fig. 5 is a schematic diagram illustrating an example of a configuration of a sports club information database according to the embodiment.
Fig. 6 is a schematic diagram illustrating an example of a configuration of an exercise history information database according to the embodiment.
Fig. 7 is a flowchart illustrating an example of a registration request process according to the embodiment.
Fig. 8 is a front view illustrating an example of a configuration of a registration screen according to the embodiment.
Fig. 9 is a front view illustrating an example of a configuration of a sports club selection screen according to the embodiment.
Fig. 10 is a front view illustrating an example of a configuration of a non-permission screen according to the embodiment.
Fig. 11 is a flowchart illustrating an example of a registration confirmation process according to the embodiment.
Fig. 12 is a flowchart illustrating an example of a medical institution confirmation process according to the embodiment.
Fig. 13 is a front view illustrating an example of a configuration of a confirmation screen according to the embodiment.
Fig. 14 is a flowchart illustrating an example of a confirmation result registration process according to the embodiment.
Fig. 15 is a flowchart illustrating an example of an enrollment process according to the embodiment.
Fig. 16 is a front view illustrating an example of a configuration of an exercise program display screen according to the embodiment.
Fig. 17 is a front view illustrating an example of a configuration of an enrollment prohibition instruction screen according to the embodiment.
Fig. 18 is a flowchart illustrating an example of an exercise execution process according to the embodiment.
Fig. 19 is a flowchart illustrating an example of an exercise status registration process according to the embodiment.
Fig. 20 is a schematic diagram illustrating an example of a configuration of an exercise program creation model according to the embodiment.

### DESCRIPTION OF EMBODIMENTS

Hereinafter, exemplary embodiments for carrying out the technique of the present disclosure will be described in detail with reference to the drawings. Note that, in the present embodiment, a case will be described in which the technique of the present disclosure is applied to an exercise support system including an exercise support device, an information reception device, and an information processing device each configured by a server computer or the like, a plurality of mobile terminals each being a terminal individually used by each patient, and a plurality of wearable terminals each being worn by each patient when performing exercise according to an exercise program. In addition, in the present embodiment, an exemplary embodiment in a case where the technique of the present disclosure is applied in a case where the patient is a patient with type 2 diabetes will be described.

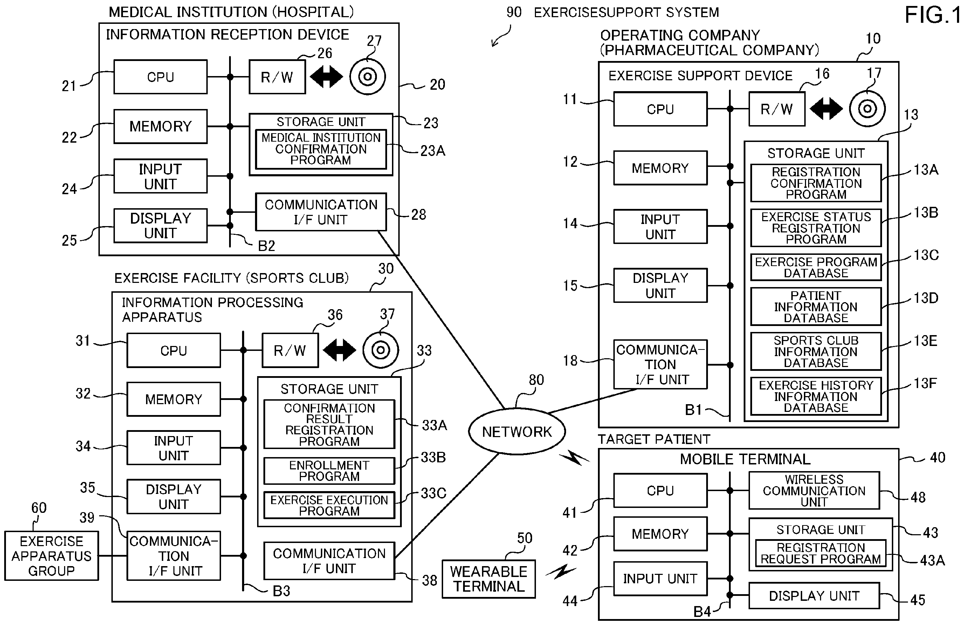

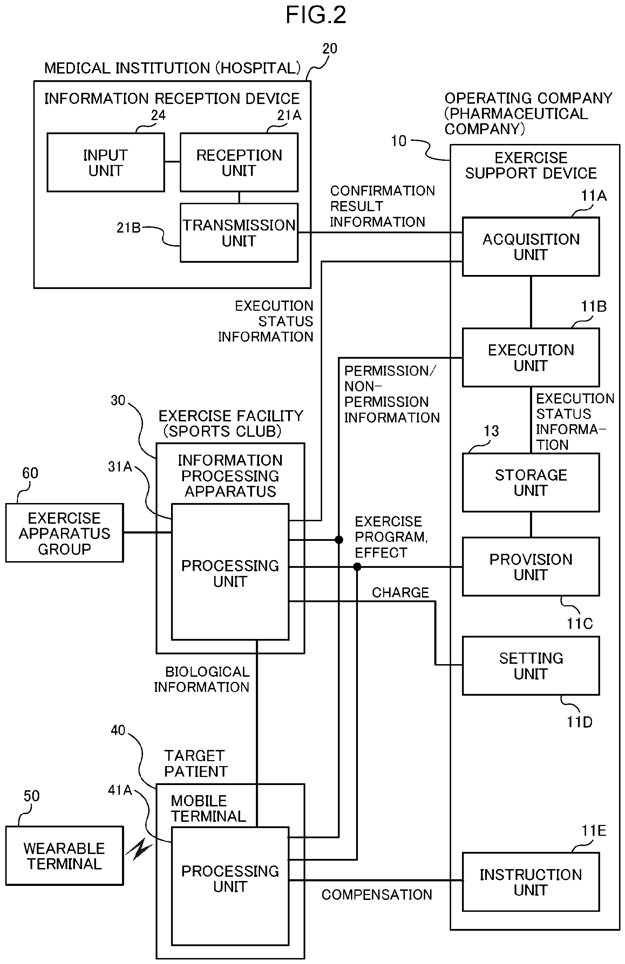

First, a configuration of an exercise support system 90 according to the present embodiment will be described with reference to Figs. 1 and 2. As illustrated in Fig. 1, an exercise support system 90 according to the present embodiment includes an exercise support device 10, an information reception device 20, and an information processing device 30, each of which can access a network 80. Note that examples of the exercise support device 10, the information reception device 20, and the information processing device 30 include general-purpose information processing devices such as a personal computer and a server computer.

In addition, the exercise support system 90 according to the present embodiment includes a plurality of mobile terminals 40 and a plurality of wearable terminals 50. Note that examples of the mobile terminals 40 include portable and wirelessly communicable terminals such as a smartphone, a tablet terminal, a personal digital assistant (PDA), and a notebook-type personal computer. Furthermore, examples of the wearable terminals 50 include terminals capable of detecting biological information, such as a wristband-type terminal, a watch-type terminal, a clip-type terminal, and a glasses-type terminal.

The exercise support device 10 according to the present embodiment is a device managed by an operating company (in the present embodiment, a pharmaceutical company; hereinafter, also simply referred to as an "operating company") of the exercise support system 90. The exercise support device 10 includes a central processing unit (CPU) 11, a memory 12 as a temporary storage area, a nonvolatile storage unit 13, an input unit 14 such as a keyboard and a mouse, a display unit 15 such as a liquid crystal display, a medium read/write device (R/W) 16, and a communication interface (I/F) unit 18. The CPU 11, the memory 12, the storage unit 13, the input unit 14, the display unit 15, the medium read/write device 16, and the communication I/F unit 18 are connected to each other via a bus B1. The medium read/write device 16 reads information written in a recording medium 17 and writes information in the recording medium 17.

The storage unit 13 is realized by a hard disk drive (HDD), a solid state drive (SSD), a flash memory, or the like. The storage unit 13 as a storage medium stores a registration confirmation program 13A and an exercise status registration program 13B. The registration confirmation program 13A and the exercise status registration program 13B are stored in the storage unit 13 when the recording medium 17 on which the registration confirmation program 13A and the exercise status registration program 13B are written is set in the medium read/write device 16 and the medium read/write device 16 reads the registration confirmation program 13A and the exercise status registration program 13B from the recording medium 17. The CPU 11 reads the registration confirmation program 13A and the exercise status registration program 13B from the storage unit 13, develops the programs in the memory 12, and sequentially executes processes included in the registration confirmation program 13A and the exercise status registration program 13B.

In addition, the storage unit 13 stores an exercise program database 13C, a patient information database 13D, a sports club information database 13E, and an exercise history information database 13F. Details of the exercise program database 13C, the patient information database 13D, the sports club information database 13E, and the exercise history information database 13F will be described later.

On the other hand, the information reception device 20 according to the present embodiment is a device managed by a medical institution (in the present embodiment, a hospital; hereinafter also simply referred to as a "medical institution") affiliated with the operating company. The information reception device 20 includes a CPU 21, a memory 22 as a temporary storage area, a nonvolatile storage unit 23, an input unit 24 such as a keyboard and a mouse, a display unit 25 such as a liquid crystal display, a medium read/write device 26, and a communication I/F unit 28. The CPU 21, the memory 22, the storage unit 23, the input unit 24, the display unit 25, the medium read/write device 26, and the communication I/F unit 28 are connected to each other via a bus B2. The medium read/write device 26 reads information written in a recording medium 27 and writes information in the recording medium 27.

The storage unit 23 is realized by an HDD, an SSD, a flash memory, or the like. The storage unit 23 as a storage medium stores a medical institution confirmation program 23A. The medical institution confirmation program 23A is stored in the storage unit 23 when the recording medium 27 on which the medical institution confirmation program 23A is written is set in the medium read/write device 26 and the medium read/write device 26 reads the medical institution confirmation program 23A from the recording medium 27. The CPU 21 reads the medical institution confirmation program 23A from the storage unit 23, develops the program in the memory 22, and sequentially executes processes included in the medical institution confirmation program 23A.

On the other hand, the information processing device 30 according to the present embodiment is a device managed by an exercise facility (in the present embodiment, sports club; hereinafter, also simply referred to as "exercise facility") affiliated with the operating company. The information processing device 30 includes a CPU 31, a memory 32 as a temporary storage area, a nonvolatile storage unit 33, an input unit 34 such as a keyboard and a mouse, a display unit 35 such as a liquid crystal display, a medium read/write device 36, a communication I/F unit 38, and a communication I/F unit 39. The CPU 31, the memory 32, the storage unit 33, the input unit 34, the display unit 35, the medium read/write device 36, the communication I/F unit 38, and the communication I/F unit 39 are connected to each other via a bus B3. The medium read/write device 36 reads information written in the recording medium 37 and writes information in the recording medium 37.

The storage unit 33 is realized by an HDD, an SSD, a flash memory, or the like. The storage unit 33 as a storage medium stores a confirmation result registration program 33A, an enrollment program 33B, and an exercise execution program 33C. The recording medium 37 in which the confirmation result registration program 33A, the enrollment program 33B, and the exercise execution program 33C are written is set in the medium read/write device 36. Then, the medium read/write device 36 reads the confirmation result registration program 33A, the enrollment program 33B, and the exercise execution program 33C from the recording medium 37. As a result, the confirmation result registration program 33A, the enrollment program 33B, and the exercise execution program 33C are stored in the storage unit 33. The CPU 31 reads the confirmation result registration program 33A, the enrollment program 33B, and the exercise execution program 33C from the storage unit 33 and develops them in the memory 32. The CPU 31 sequentially executes processes included in the confirmation result registration program 33A, the enrollment program 33B, and the exercise execution program 33C.

Meanwhile, the exercise program according to the present embodiment is a program by combining aerobic exercise and resistance exercise, and the exercise facility according to the present embodiment is provided with a plurality of types of exercise apparatus to be used when performing aerobic exercise and resistance exercise. For example, examples of the exercise apparatus used when performing the aerobic exercise include a treadmill and a motorcycle. In addition, examples of the exercise apparatus used when performing the resistance exercise include a chest press, a pull-down, a leg press, and the like.

Then, these exercise apparatus (hereinafter, referred to as an "exercise apparatus group") 60 according to the present embodiment have a function of measuring and transmitting a physical quantity (hereinafter, referred to as "execution status information") indicating an execution status of an exercise by the corresponding exercise apparatus. Therefore, the exercise apparatus group 60 is connected to the communication I/F unit 39, and the information processing device 30 can receive the execution status information from the exercise apparatus group 60.

In the present embodiment, an intensity, duration, and type of the aerobic exercise and the resistance exercise included in the exercise program are considered to be defined, but the present invention is not limited thereto. For example, one of the frequency, the intensity, the duration, and the type, or a plurality of types of combinations excluding the combination of the intensity, the duration, and the type may be defined in the aerobic exercise and the resistance exercise.

Furthermore, in the present embodiment, a program whose usefulness has been confirmed by a predetermined method is applied as the exercise program. In the present embodiment, as the predetermined method, a method for verifying usefulness by a parallel group comparison test of an exercise intervention group and a control group is applied. This makes it possible to verify scientific usefulness of the exercise program itself.

On the other hand, the mobile terminals 40 according to the present embodiment are terminals owned by each of a plurality of patients (in the present embodiment, for example, patients with type 2 diabetes; hereinafter, also simply referred to as "target patients") who visit the medical institution and are expected to use a service (hereinafter, referred to as a "provided service") provided by the exercise support system 90. The mobile terminals 40 include a CPU 41, a memory 42 as a temporary storage area, a nonvolatile storage unit 43, an input unit 44 such as a touch panel, a display unit 45 such as a liquid crystal display, and a wireless communication unit 48. The CPU 41, the memory 42, the storage unit 43, the input unit 44, the display unit 45, and the wireless communication unit 48 are connected to each other via a bus B4.

The storage unit 43 is realized by an HDD, an SSD, a flash memory, or the like. The storage unit 43 as a storage medium stores a registration request program 43A. The registration request program 43A is installed in the storage unit 43 in advance. The CPU 41 reads the registration request program 43A from the storage unit 43, develops the registration request program in the memory 42, and sequentially executes processes included in the registration request program 43A.

Furthermore, the wearable terminals 50 according to the present embodiment are worn by each of the target patients when the target patient performs an exercise by an exercise program. Note that, although not illustrated, each of the wearable terminals 50 according to the present embodiment has a function of measuring biological information including a heart rate of a wearer (patient) and a function of wirelessly transmitting the measured biological information to a mobile terminal 40 possessed by the wearer.

Next, functional configurations of the exercise support device 10, the information reception device 20, the information processing device 30, and the mobile terminal 40 according to the present embodiment will be described with reference to Fig. 2. As illustrated in Fig. 2, the exercise support device 10 according to the present embodiment includes an acquisition unit 11A, an execution unit 11B, a provision unit 11C, a setting unit 11D, and an instruction unit 11E. The CPU 11 of the exercise support device 10 executes the registration confirmation program 13A and the exercise status registration program 13B to function as the acquisition unit 11A, the execution unit 11B, the provision unit 11C, the setting unit 11D, and the instruction unit 11E.

The acquisition unit 11A according to the present embodiment acquires, from the medical institution, confirmation result information indicating a confirmation result regarding the execution of an exercise by a patient attending the medical institution at an exercise facility in which the exercise according to the exercise program based on a disease of the patient is performed. In the present embodiment, the following two check points by a doctor in charge of the corresponding patient are applied as the check points to be confirmed by the medical institution.

Check point 1: check that the patient is a patient recommended by a person who provides exercise according to the exercise program with use of the provided service.

Check point 2: confirm that the patient is not a patient to whom the provided service cannot be recommend in clinical practice.

Note that the above-described check point 1 is intended to avoid an application due to some kind of mistake or mischief. In addition, the purpose of the check point 2 is to avoid an occurrence of a situation in which the exercise with use of the provided service does not contribute to the improvement of a disease state of the patient or a situation in which the exercise adversely affects the patient.

However, the confirmation of the check point by the medical institution is not limited in this form, and for example, other confirmation such as checking that the patient does not change the intensity of the exercise according to the exercise program provided in advance without permission, may be applied. In addition, a person who performs the confirmation is not limited to the doctor in charge, and for example, a person related to a medical institution where the patient visits, such as another doctor, a nurse, or a physical therapist instructed by the doctor in charge, may perform the confirmation.

Then, on the basis of the confirmation result information acquired by the acquisition unit 11A, the execution unit 11B executes a process of transmitting permission or non-permission information of execution of the exercise according to the exercise program of the patient at the exercise facility to both the exercise facility and the patient. More specifically, the execution unit 11B transmits permission information in a case where the confirmation result information is information indicating that there is no problem, or transmits non-permission information in a case where the confirmation result information is information indicating that there is a problem, to both the exercise facility and the patient. Note that the transmission destination of the permission information or the non-permission information is not necessarily to both the exercise facility and the patient, and may be, for example, a form in which the permission information or the non-permission information is transmitted to only one of the exercise facility and the patient.

On the other hand, the provision unit 11C provides exercise program information indicating the exercise program to both the exercise facility and the patient. Note that the destination of the exercise program information is not limited to the two locations of the exercise facility and the patient. The exercise program information may be provided to any one place of a medical institution, an exercise facility, and a patient, or a combination of a plurality of places excluding the combination of two places of the exercise facility and the patient. Note that, in a case where the destination of providing the exercise program information is one or two of the medical institution, the exercise facility, and the patient, the exercise program information may be provided from the providing destination to the non-providing destination.

In addition to the exercise program information, the provision unit 11C provides both the exercise facility and the patient with effect information indicating effect of the exercise according to the exercise program information. Note that the destination of the effect information is not limited to the two locations of the exercise facility and the patient. The destination of the effect information may be any one place of the medical institution, the exercise facility, and the patient, or a combination of a plurality of places excluding the combination of two places of the exercise facility and the patient. Note that, in a case where the destination of the effect information is one or two of the medical institution, the exercise facility, and the patient, the form of providing the effect information from the provision destination to the non-provision destination may be adopted, which is similar to the case of the exercise program information.

In addition, the setting unit 11D sets a charge corresponding to the use of the exercise facility by the patient as compensation for the provision of the exercise program information by the provision unit 11C. Here, in the present embodiment, the amount of the charge includes at least a part of cost for facility use received by the exercise facility from the patient. Then, the instruction unit 11E instructs the patient to pay compensation for the provision of the exercise program information. However, the present invention is not limited to these forms, and a form in which either one of the setting of the charge for the exercise facility by the setting unit 11D and the instruction of the payment of the compensation to the patient by the instruction unit 11E is not executed or a form in which both are not executed may be adopted.

On the other hand, the acquisition unit 11A further acquires the above-described execution status information indicating the execution status of the exercise according to the exercise program of the patient, and the execution unit 11B further executes a process of storing the execution status information acquired by the acquisition unit 11A in the storage unit 13 so that a medical worker of the medical institution can refer to the execution status information. Here, the acquisition unit 11A according to the present embodiment acquires, as the execution status information, the above-described biological information transmitted from the wearable terminal 50 worn by the patient during exercise. Note that, in the present embodiment, biological information including the heart rate of the patient measured by the wearable terminal 50 is transmitted to the information processing device 30 via the mobile terminal 40 possessed by the patient. Then, the information processing device 30 transmits the execution status information received from the exercise apparatus group 60 to the exercise support device 10 so as to include the biological information of the corresponding patient.

On the other hand, the information reception device 20 according to the present embodiment includes a reception unit 21A and a transmission unit 21B. The CPU 21 of the information reception device 20 executes the medical institution confirmation program 23A to function as the reception unit 21A and the transmission unit 21B.

The reception unit 21A according to the present embodiment receives the above-described confirmation result information. Then, the transmission unit 21B according to the present embodiment transmits the confirmation result information received by the reception unit 21A to the exercise support device 10.

Furthermore, the information processing device 30 according to the present embodiment includes a processing unit 31A. The CPU 31 of the information processing device 30 functions as the processing unit 31A by executing the confirmation result registration program 33A, the enrollment program 33B, and the exercise execution program 33C.

The processing unit 31A according to the present embodiment performs processing or the like corresponding to the process of transmitting permission or non-permission information of the exercise according to the exercise program of the patient at the exercise facility by the execution unit 11B in the exercise support device 10. The processing unit 31A according to the present embodiment performs, as the processing, each process of confirmation result registration processing, enrollment processing, and exercise execution processing to be described later, and details thereof will be described later.

Furthermore, the mobile terminal 40 according to the present embodiment includes a processing unit 41A. The CPU 41 of the mobile terminal 40 executes the registration request program 43A to function as the processing unit 41A.

The processing unit 41A according to the present embodiment performs a registration request process to be described later, a transmitting process of the above-described biological information to the information processing device 30, and the like.

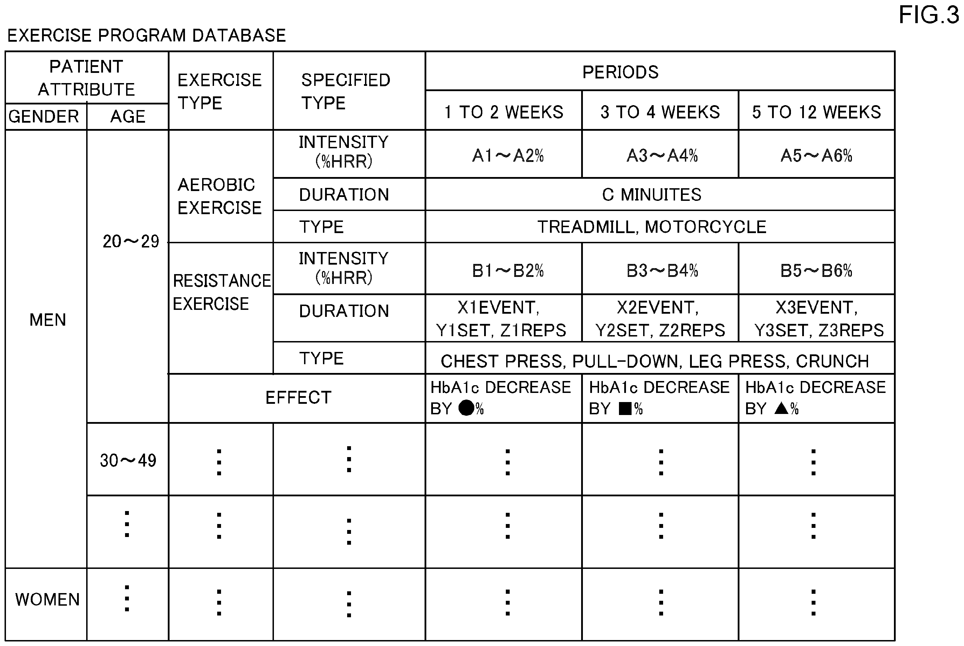

Next, the exercise program database 13C according to the present embodiment will be described with reference to Fig. 3.

As illustrated in Fig. 3, the exercise program database 13C according to the present embodiment stores the exercise program information including a combination of each exercise type of aerobic exercise and resistance exercise for each patient attribute. In the example illustrated in Fig. 3, a specified type (In the present embodiment, intensity, duration, and type) for each predetermined period (in the example illustrated in Fig. 3, a period of three stages of 1 to 2 weeks (hereinafter, referred to as a "first period"), 3 to 4 weeks (hereinafter, referred to as a "second period"), and 5 to 12 weeks (hereinafter, referred to as a "third period")) is stored as the exercise program information.

In the example illustrated in Fig. 3, for example, as exercise program information corresponding to men from 20 to 29 years of age, regarding the aerobic exercise, A1 to A 2% is applied as the intensity in the first period, C minutes is applied as the duration, and a treadmill, a motorcycle, or the like is applied as the type. In the present embodiment, as the intensity in the aerobic exercise, the ratio of time during which the heart rate of a target patient is within a predetermined range as the target heart rate to the duration is applied. In the present embodiment, as the intensity of the resistance exercise, the ratio of a maximum liftable weight (the maximum weight (load) that can be lifted up only once) to an estimated value is applied. However, it goes without saying that the intensity of each of the aerobic exercise and the resistance exercise is not limited to these forms.

In addition, as illustrated in Fig. 3, the exercise program database 13C according to the present embodiment also stores the above-described effect information indicating the effect in a case where each exercise is performed. In the example illustrated in Fig. 3, a reduction rate of hemoglobin A1c (HbA1c) is applied as the effect information, but it goes without saying that the present invention is not limited thereto. Note that, as described above, the exercise program information registered in the exercise program database 13C according to the present embodiment has already been confirmed to be useful.

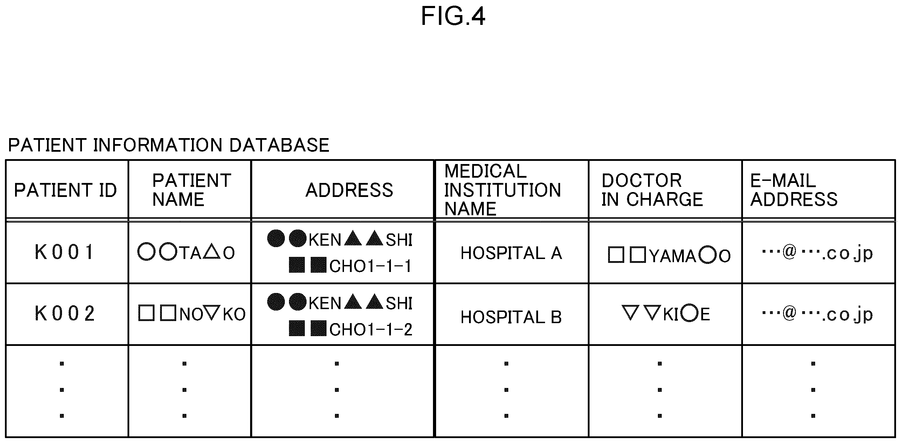

Next, the patient information database 13D according to the present embodiment will be described with reference to Fig. 4.

As illustrated in Fig. 4, the patient information database 13D according to the present embodiment stores each piece of information of a patient identification (ID), a patient name, an address, a medical institution name, a doctor in charge, and an e-mail address.

The patient ID is information assigned in advance for each target patient in order to identify each target patient, the patient name is information indicating the name of the corresponding target patient, and the address is information indicating the address of the corresponding target patient. In addition, the medical institution name is information indicating the name of the medical institution that the corresponding target patient visits, the doctor in charge is information indicating the name of the doctor in charge of the corresponding target patient, and the e-mail address is information indicating the e-mail address of the doctor in charge.

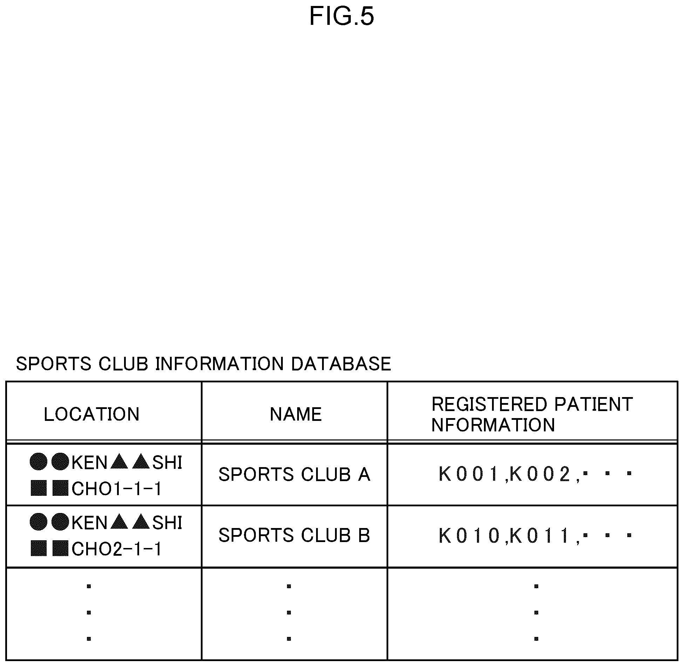

Next, a sports club information database 13E according to the present embodiment will be described with reference to Fig. 5.

As illustrated in Fig. 5, the sports club information database 13E according to the present embodiment stores each piece of information of a location, a name, and registered patient information for each sports club supported by the exercise support system 90.

The location is information indicating a location of a corresponding sports club, the name is information indicating a name of the corresponding sports club, and the registered patient information is information indicating a patient ID of a target patient permitted to use a provided service in the corresponding sports club.

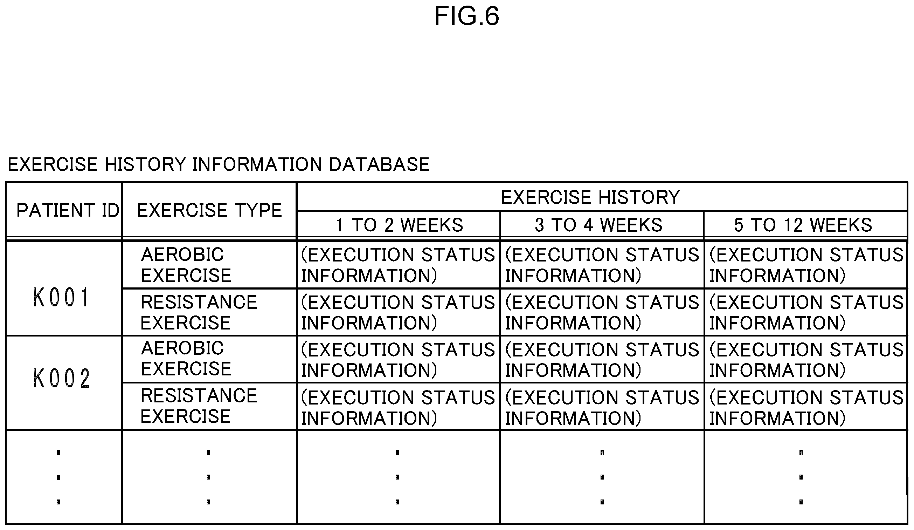

Next, the exercise history information database 13F according to the present embodiment will be described with reference to Fig. 6.

As illustrated in Fig. 6, the exercise history information database 13F according to the present embodiment stores each piece of information of a patient ID, an exercise type, and an exercise history for each of the predetermined periods (in the present embodiment, the three stages of the first period, the second period, and the third period described above).

The patient ID is the same information as the patient ID in the patient information database 13D, and the exercise type is the same information as the exercise type in the exercise program database 13C. Furthermore, the exercise history is information indicating the actual exercise status of the corresponding target patient in each of the predetermined periods, and in the present embodiment, the above-described execution status information is applied as the information.

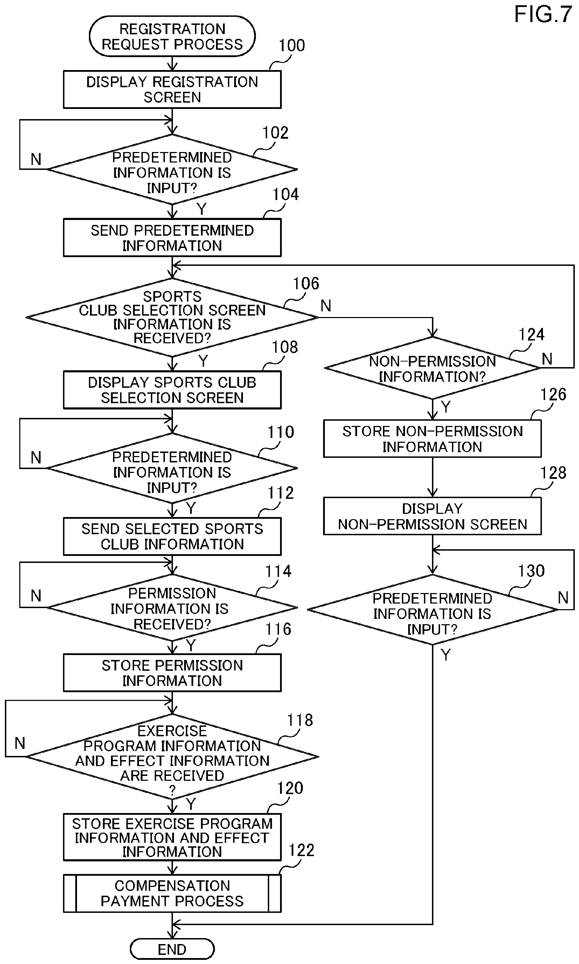

Next, the operation of the exercise support system 90 according to the present embodiment will be described with reference to Figs. 7 to 19. Note that, in order to avoid complication, a case where each database of the exercise program database 13C and the patient information database 13D is constructed in advance will be described as follows. Furthermore, in order to avoid complication, a case where each piece of information of the location and the name of the sports club information database 13E is registered in advance, and each piece of information of the patient ID and the exercise type of the exercise history information database 13F is registered in advance will be described as follows.

First, the operation of the mobile terminal 40 according to the present embodiment will be described with reference to Figs. 7 to 10. When the CPU 41 of any one of plurality of mobile terminals 40 executes the registration request program 43A, the registration request process illustrated in Fig. 7 is executed. The registration request process illustrated in Fig. 7 is executed, for example, when an execution instruction of the registration request process is input from a patient who has a medical examination at a medical institution corresponding to the exercise support system 90 via the input unit 44 of the mobile terminal 40 of the target patient.

In Step 100 of Fig. 7, the processing unit 41A performs control to display a registration screen having a predetermined configuration on the display unit 45, and then in Step 102, the processing unit 41A waits until predetermined information is input.

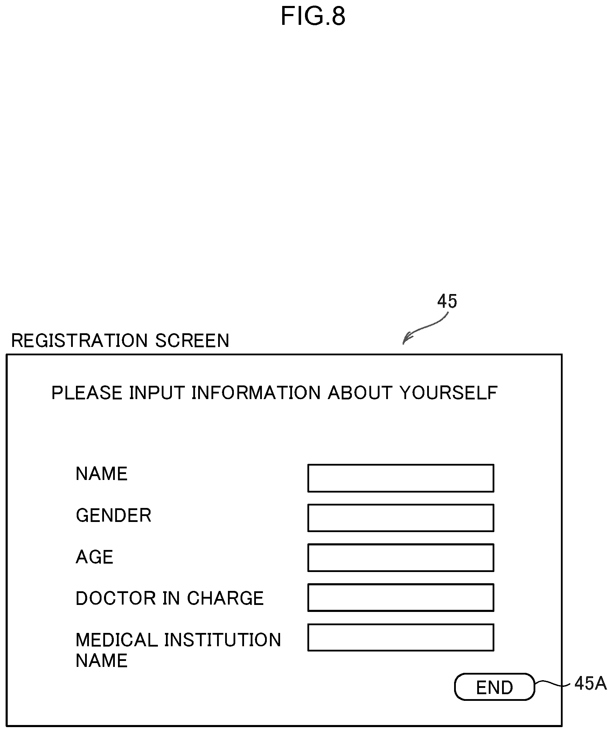

Fig. 8 illustrates an example of a registration screen according to the present embodiment. As illustrated in Fig. 8, on the registration screen according to the present embodiment, a message prompting input of information regarding the patient himself/herself is displayed. Furthermore, on the registration screen according to the present embodiment, an input region for inputting a name, gender, and age of the patient, a name of the doctor in charge, and a name of the medical institution received by the patient is displayed.

When the registration screen illustrated in Fig. 8 is displayed on the display unit 45, the patient inputs each piece of information regarding the patient to the corresponding input area via the input unit 44, and then designates an end button 45A via the input unit 44. If the end button 45A is designated by the patient, an affirmative determination is made in Step 102, and the process proceeds to Step 104.

In Step 104, the processing unit 41A transmits each piece of information (hereinafter, referred to as "registration information") input by the patient on the registration screen to the exercise support device 10 via the wireless communication unit 48. Upon receiving the registration information, the exercise support device 10 transmits the sports club selection screen information or the non-permission information to the mobile terminal 40 as a transmission source of the registration information as described later.

Therefore, in Step 106, the processing unit 41A determines whether or not the sports club selection screen information has been received from the exercise support device 10, and proceeds to Step 108 if the determination is affirmative. In Step 108, the processing unit 41A performs control to display a sports club selection screen indicated by the sports club selection screen information received from the exercise support device 10 on the display unit 45, and then in Step 110, the processing unit 41A waits until predetermined information is input.

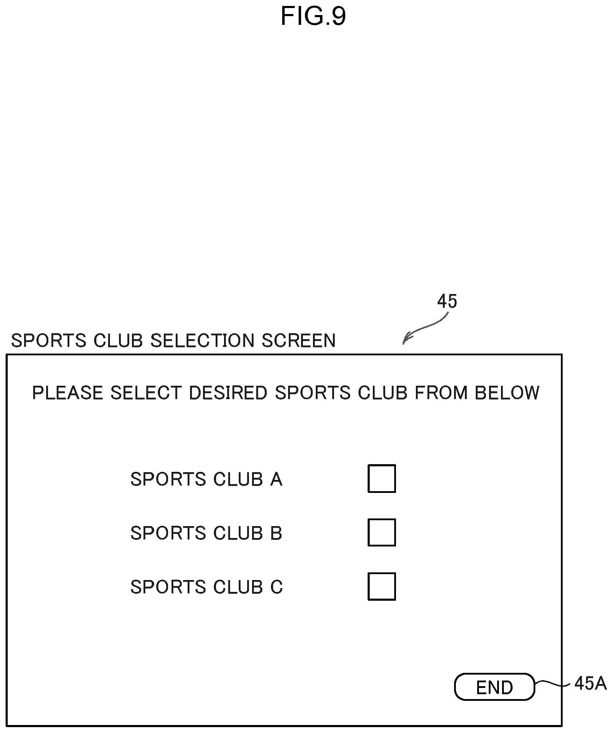

Fig. 9 illustrates an example of a sports club selection screen according to the present embodiment. As illustrated in Fig. 9, on the sports club selection screen according to the present embodiment, a message prompting selection of a desired sports club is displayed. Furthermore, on the sports club selection screen according to the present embodiment, the name of a sports club targeted by the exercise support system 90 and a designated area to be designated when selecting a corresponding sports club are displayed.

When the sports club selection screen illustrated in Fig. 9 is displayed on the display unit 45, the patient designates a designated area corresponding to a sports club to which the patient wants to visit via the input unit 44, and then designates the end button 45A via the input unit 44. If the end button 45A is designated by the patient, an affirmative determination is made in Step 110, and the process proceeds to Step 112.

In Step 112, the processing unit 41A transmits information (hereinafter, referred to as "selected sports club information") indicating the sports club selected by the patient on the sports club selection screen to the exercise support device 10 via the wireless communication unit 48. Upon receiving the selected sports club information, the exercise support device 10 transmits the permission information to the mobile terminal 40 that is the transmission source of the selected sports club information, and then transmits the exercise program information and the effect information to the mobile terminal 40 as to be described later.

Therefore, in Step 114, the processing unit 41A waits until permission information is received from the exercise support device 10, and in Step 116, the processing unit 41A stores the permission information received from the exercise support device 10 in the storage unit 43. In Step 118, the processing unit 41A waits until the exercise program information and the effect information are received from the exercise support device 10, and in Step 120, the processing unit 41A stores the exercise program information and the effect information received from the exercise support device 10 in the storage unit 43. By storing the exercise program information and the effect information, the patient can refer to an exercise program and an effect indicated by the exercise program information and the effect information at any time.

By the way, after transmitting the exercise program information and the effect information to the mobile terminal 40, the exercise support device 10 according to the present embodiment executes a process (hereinafter, referred to as "compensation payment instruction process") of instructing the payment of the compensation for the provision of the exercise program information to the patient by the instruction unit 11E as described above. Therefore, in Step 122, the processing unit 41A executes the compensation payment instruction process to the operating company according to the compensation payment instruction process, and thereafter, ends the present registration request process.

On the other hand, if a negative determination is made in Step 106, the process proceeds to Step 124, and the processing unit 41A determines whether non-permission information is received from the exercise support device 10. If a negative determination is made, the process returns to Step 106, and if an affirmative determination is made, the process proceeds to Step 126.

In Step 126, the processing unit 41A stores the non-permission information received from the exercise support device 10 in the storage unit 43. In Step 128, the processing unit 41A performs control to display a non-permission screen having a predetermined configuration on the display unit 45, and then in Step 130, the processing unit 41A waits until predetermined information is input.

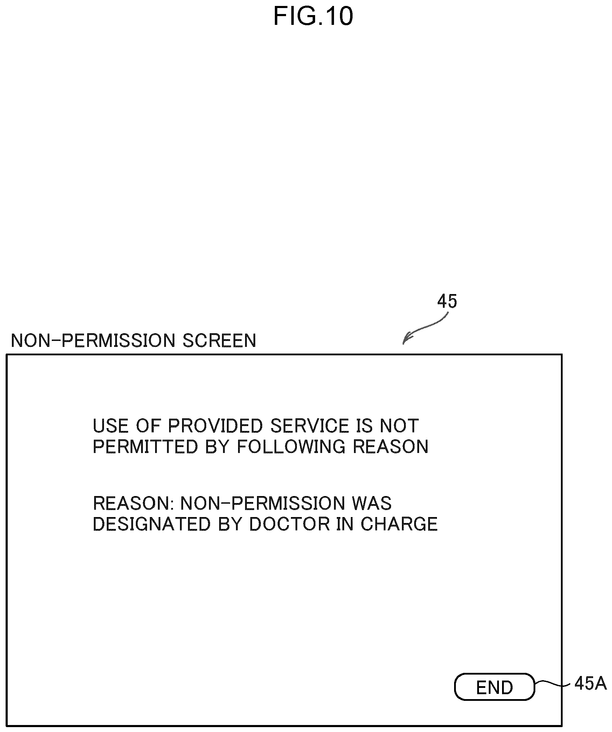

Fig. 10 illustrates an example of a non-permission screen according to the present embodiment. As illustrated in Fig. 10, on the non-permission screen according to the present embodiment, the fact that the use of the provided service is not permitted is displayed together with the reason. When the non-permission screen illustrated in Fig. 10 is displayed on the display unit 45, the patient recognizes that the provided service is unavailable, and then designates the end button 45A via the input unit 44. If the end button 45A is designated by the patient, an affirmative determination is made in Step 130, and the present registration request process ends.

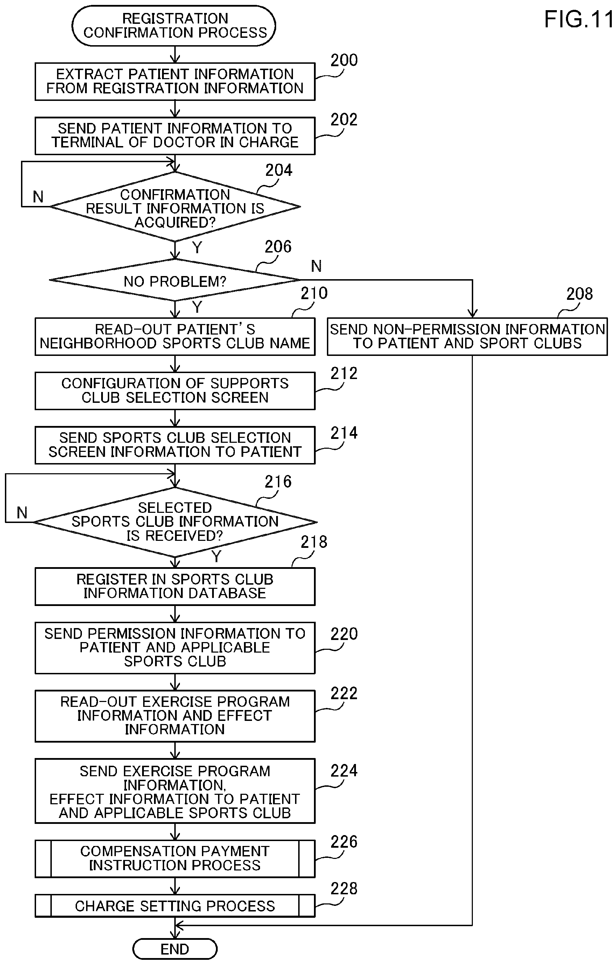

Next, the operation of the exercise support device 10 according to the present embodiment will be described with reference to Fig. 11. When the CPU 11 of the exercise support device 10 executes the registration confirmation program 13A, a registration confirmation process illustrated in Fig. 11 is executed. The registration confirmation process illustrated in Fig. 11 is executed in a case where the registration information described above is received from the mobile terminal 40 possessed by any patient.

In Step 200 of Fig. 11, the acquisition unit 11A extracts, from the registration information received from the mobile terminal 40, respective pieces of information (hereinafter, referred to as "patient information") of the name, gender, and age of the patient who has transmitted the registration information. In Step 202, the acquisition unit 11A reads the e-mail address of the doctor in charge corresponding to the doctor name in charge included in the received registration information from the patient information database 13D, and transmits the extracted patient information to the read e-mail address by e-mail. By the transmission of the e-mail, the information reception device 20 used by the doctor in charge indicated by the doctor name in charge input by the patient receives the patient information corresponding to the patient. Upon receiving the patient information, the information reception device 20 transmits confirmation result information to the exercise support device 10 as to be described later.

Therefore, in Step 204, the acquisition unit 11A waits until the confirmation result information is received from the information reception device 20. In Step 206, the execution unit 11B determines whether the confirmation result information received from the information reception device 20 is information indicating that there is no problem (in the present embodiment, information indicating "permission"), and proceeds to Step 208 if a negative determination (in the present embodiment, if the confirmation result information is information indicating "not permitted").

In Step 208, the execution unit 11B transmits the non-permission information described above to the mobile terminal 40 that is the transmission source of the registration information and all the sports clubs targeted by the exercise support system 90 via the communication I/F unit 18, and then ends the present registration confirmation process. Here, when transmitting the non-permission information to all the sports clubs described above, the execution unit 11B also transmits information indicating the name of the patient in the patient information.

On the other hand, if an affirmative determination is made in Step 206, that is, if the confirmation result information received from the information reception device 20 is information indicating that there is no problem, the process proceeds to Step 210. In Step S210, the execution unit 11B reads information indicating the name of a nearby sports club of the corresponding patient as to be described below.

That is, first, the execution unit 11B reads information indicating an address corresponding to the name of the patient in the received patient information from the patient information database 13D. Then, the execution unit 11B reads, from the sports club information database 13E, the name of a sport club (In the present embodiment, sports clubs having the same city name in a location are provided.) in which the neighborhood of the read address is a location.

In Step 212, the execution unit 11B configures the above-described sport club selection screen information using the name of the sport club read by the processing in Step 210. In Step 214, the execution unit 11B transmits the configured sports club selection screen information to the mobile terminal 40 as a transmission source of the registration information via the communication I/F unit 18.

Upon receiving the sports club selection screen information, the mobile terminal 40 that is the transmission source of the registration information transmits the selected sports club information indicating the sports club selected by the corresponding patient among the sports clubs displayed on the sports club selection screen, as described above. Therefore, in Step 216, the execution unit 11B waits until the selected sports club information is received from the mobile terminal 40 of the patient.

In Step 218, the execution unit 11B reads the patient ID corresponding to the name of the patient in the received patient information from the patient information database 13D. Then, the execution unit 11B registers the read patient ID in the sports club information database 13E as registered patient information corresponding to the sports club indicated by the selected sports club information.

In Step 220, the execution unit 11B transmits the permission information described above to the mobile terminal 40 that is the transmission source of the registration information and the sports club (hereinafter, referred to as "applicable sports club") indicated by the selected sports club information via the communication I/F unit 18. Here, when transmitting the permission information to the applicable sports club, the execution unit 11B also transmits information indicating the name of the patient in the patient information. In Step 222, the provision unit 11C reads the exercise program information and the effect information corresponding to the gender and the age of the patient in the patient information from the exercise program database 13C.

In Step 224, the provision unit 11C transmits the read exercise program information and effect information to the mobile terminal 40 that is the transmission source of the registration information and the applicable sports club via the communication I/F unit 18.

In Step 226, the instruction unit 11E executes the compensation payment instruction process described above. In Step 228, the setting unit 11D performs a charge setting process of setting a charge corresponding to the use of the applicable sport club by the patient as compensation for the provision of the exercise program information to the applicable sport club, and thereafter, ends the present registration confirmation process. Note that, as described above, in the present embodiment, the amount of the charge includes at least a part of the cost for facility use received by the exercise facility from the patient, but it goes without saying that the amount of the charge is not limited to this.

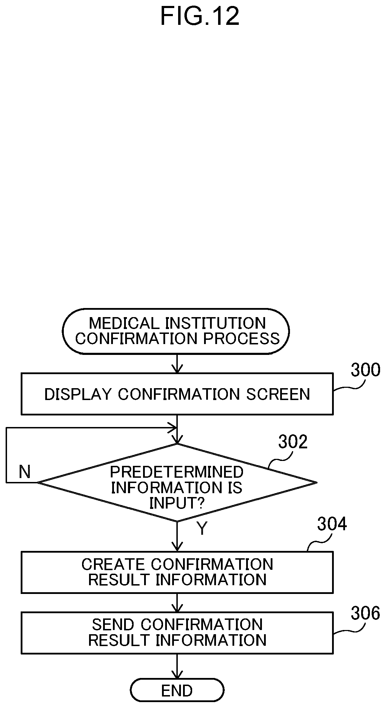

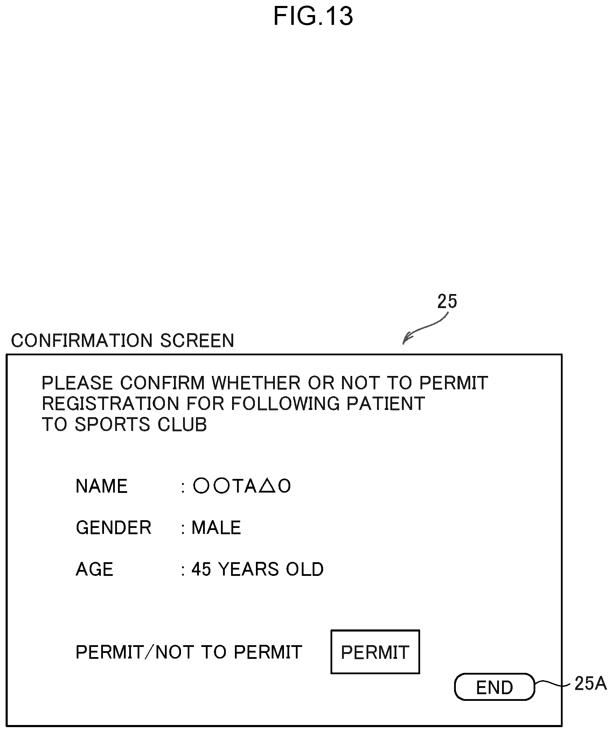

Next, the operation of the information reception device 20 according to the present embodiment will be described with reference to Figs. 12 and 13. The CPU 21 of the information reception device 20 executes the medical institution confirmation program 23A, whereby the medical institution confirmation process illustrated in Fig. 12 is executed. The medical institution confirmation process illustrated in Fig. 12 is executed when the above-described patient information is received from the exercise support device 10.

In Step 300 of Fig. 12, the reception unit 21A configures confirmation screen information indicating a confirmation screen having a predetermined configuration using the received patient information, and performs control to display the confirmation screen on the display unit 25. Thereafter, in Step 302, the reception unit 21A waits until predetermined information is input.

Fig. 13 illustrates an example of a confirmation screen according to the present embodiment. As illustrated in Fig. 13, on the confirmation screen according to the present embodiment, a message prompting confirmation as to whether or not registration of the patient to a sports club is permitted is displayed, and patient information (In the present embodiment, a name, a gender, and an age are used.) of the patient is displayed. Furthermore, on the confirmation screen according to the present embodiment, an input region for inputting whether or not to permit registration of a patient to a sports club is displayed.

When the confirmation screen illustrated in Fig. 13 is displayed on the display unit 25, the doctor in charge of the corresponding patient inputs information indicating "permitted" or "not permitted" to the input region via the input unit 24, and then designates an end button 25A via the input unit 24. When the end button 25A is designated by the doctor in charge, an affirmative determination is made in Step 302, and the process proceeds to Step 304.

In Step 304, the transmission unit 21B creates the above-described confirmation result information by using the information indicating "permitted" or "not permitted" input by the doctor in charge on the confirmation screen. In Step 306, the transmission unit 21B transmits the created confirmation result information to the exercise support device 10, and thereafter, ends the present medical institution confirmation process.

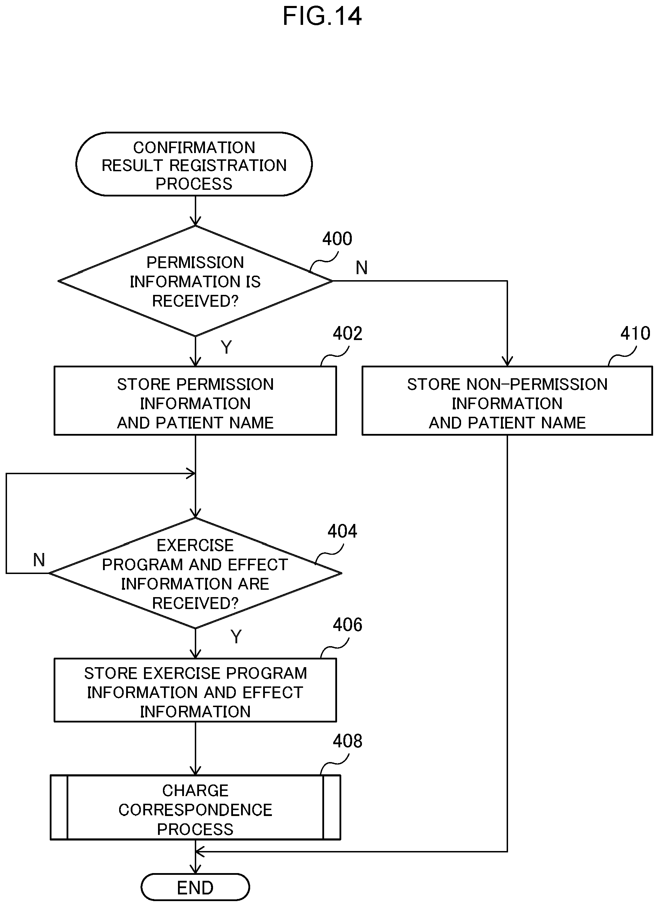

Next, the operation of the information processing device 30 according to the present embodiment will be described with reference to Figs. 14 to 18. First, the operation of the information processing device 30 when the confirmation result registration process is executed will be described with reference to Fig. 14. The CPU 31 of the information processing device 30 executes the confirmation result registration program 33Ato execute the confirmation result registration process illustrated in Fig. 14. The confirmation result registration process illustrated in Fig. 14 is executed when the permission information or the non-permission information described above is received from the exercise support device 10.

In Step 400 of Fig. 14, the processing unit 31A determines whether or not the received information is permission information, and if an affirmative determination is made, the process proceeds to Step 402, and the processing unit 31A stores the received permission information and the information indicating the name of the patient (hereinafter, referred to as "name information") received together with the permission information in the storage unit 33 in association with each other.

As described above, the exercise support device 10 transmits the permission information to the information processing device 30 of the applicable sports club, and then transmits the exercise program information and the effect information to the information processing device 30.

Therefore, in Step 404, the processing unit 31A waits until receiving the exercise program information and the effect information from the exercise support device 10. In Step 406, the processing unit 31A stores the received exercise program information and effect information in the storage unit 33 in association with the permission information and the name information stored in the storage unit 33 in the processing in Step 402 performed immediately before.

In Step 408, the processing unit 31A executes the compensation payment instruction process to the operating company according to the charge setting process by the exercise support device 10 described above, and thereafter, ends the present confirmation result registration process.

On the other hand, if a negative determination is made in Step 400, that is, when the received information is the non-permission information, the process proceeds to Step 410. In Step 410, the processing unit 31A stores the received non-permission information and the name information received together with the non-permission information in association with the storage unit 33, and then ends the present confirmation result registration process.

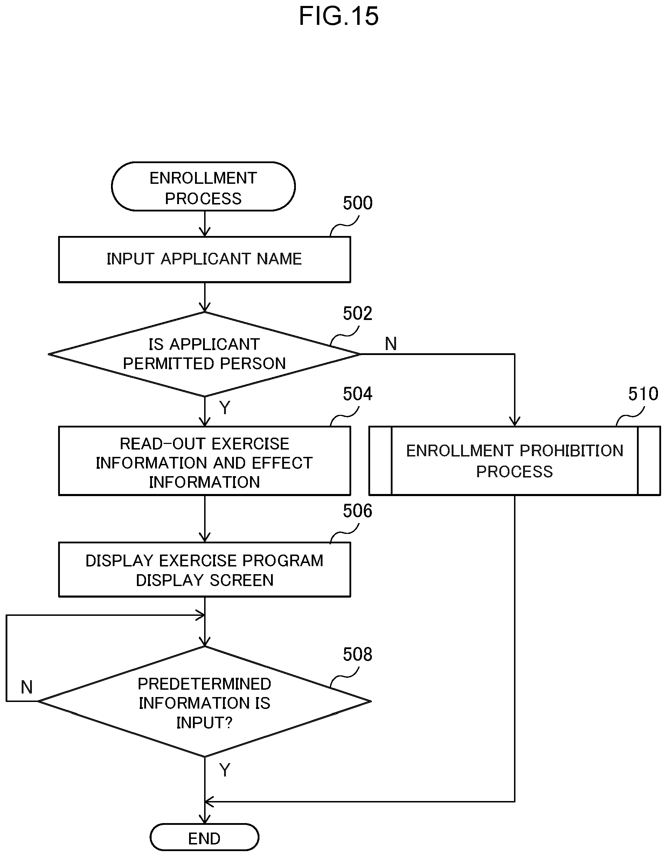

Next, an operation of the information processing device 30 when the enrollment process is executed will be described with reference to Fig. 15. When the CPU 31 of the information processing device 30 executes the enrollment program 33B, the enrollment process illustrated in Fig. 15 is executed. The enrollment process illustrated in Fig. 15 is executed when an instruction to execute the enrollment process is input from the person in charge of the applicable sports club via the input unit 34 in response to the application for use of the provided service. When accepting the application for use of the provided service, the person in charge of the sports club (hereinafter, simply referred to as "person in charge") causes an applicant to write his/her name in a predetermined document.

In Step 500 of Fig. 15, the processing unit 31A receives an input via the input unit 34 by the person in charge with a name described in the document by the applicant. In Step 502, the processing unit 31A determines whether the name information indicating the accepted name of the applicant is stored in the storage unit 33 in association with the permission information, thereby determining whether the applicant is a person permitted by the doctor in charge. When an affirmative determination is made, the process proceeds to Step 504.

In Step 504, the processing unit 31A reads the exercise program information and the effect information stored in association with the name information indicating the name of the applicant from the storage unit 33. In step 506, the processing unit 31A performs control to display an exercise program display screen having a predetermined configuration on the display unit 35 using the read exercise program information and effect information. Thereafter, in Step 508, the processing unit 31A waits until predetermined information is input.

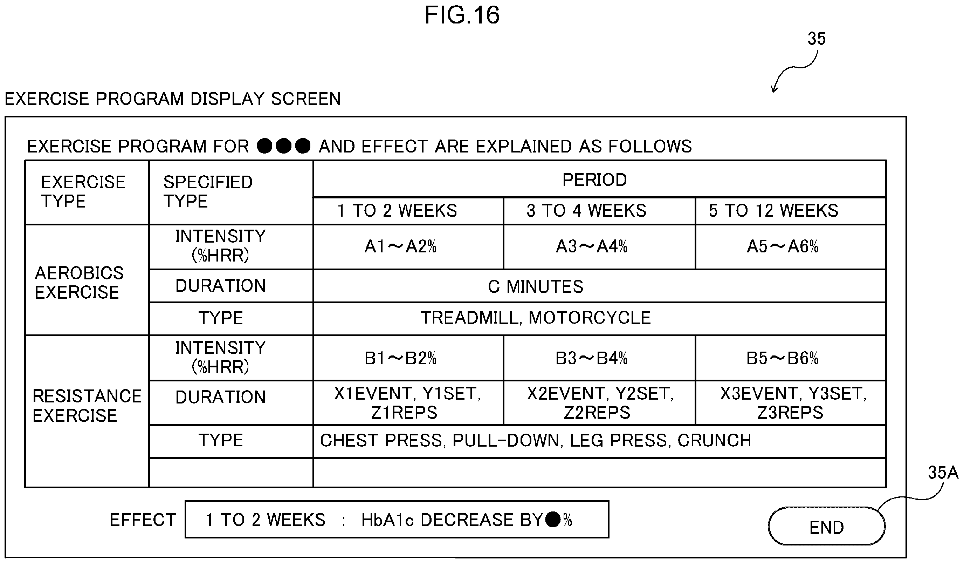

Fig. 16 illustrates an example of an exercise program display screen according to the present embodiment. As illustrated in Fig. 16, on the exercise program display screen according to the present embodiment, the exercise program indicated by the exercise program information suitable for the applicant (target patient) and the effect by the exercise program indicated by the effect information are displayed.

When the exercise program display screen illustrated in Fig. 16 is displayed on the display unit 35, the person in charge specifies the end button 35A via the input unit 34 after confirming the exercise program for the applicant and the effect thereof. If the end button 35A is designated by the person in charge, an affirmative determination is made in Step 508, and the present enrollment process ends. When the exercise program display screen is displayed on the display unit 35, the person in charge performs an enrollment procedure for the applicant.

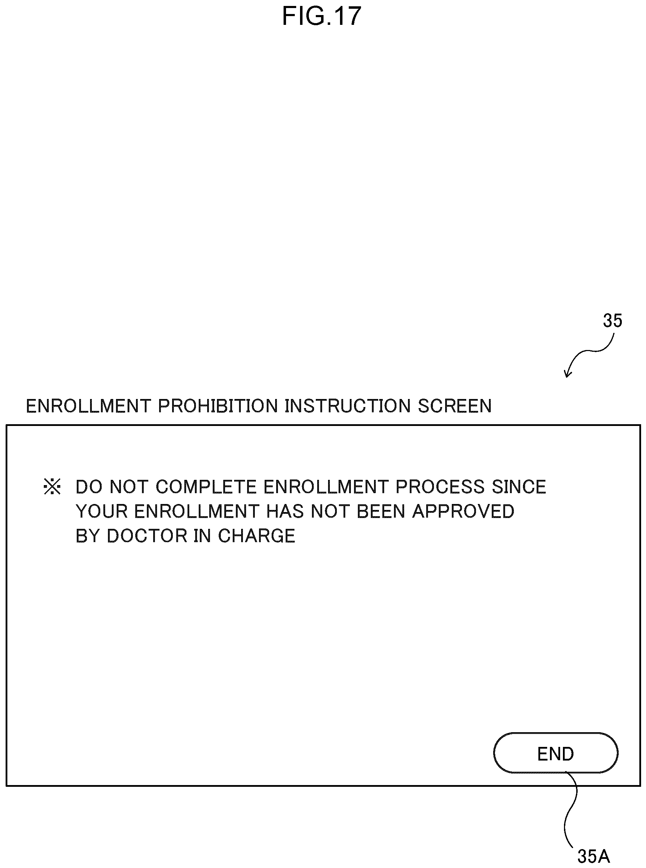

On the other hand, if a negative determination is made in Step 502, the process proceeds to Step 510, and the processing unit 31A executes a predetermined process (hereinafter, referred to as "enrollment prohibition process") for prohibiting the enrollment of the applicant, and thereafter, ends the present enrollment process. In the present embodiment, as the enrollment prohibition process, a process of displaying an enrollment prohibition instruction screen indicating that the enrollment in the provided service is prohibited, as illustrated in Fig. 17 by the display unit 35 is applied as an example, but the process is not limited thereto.

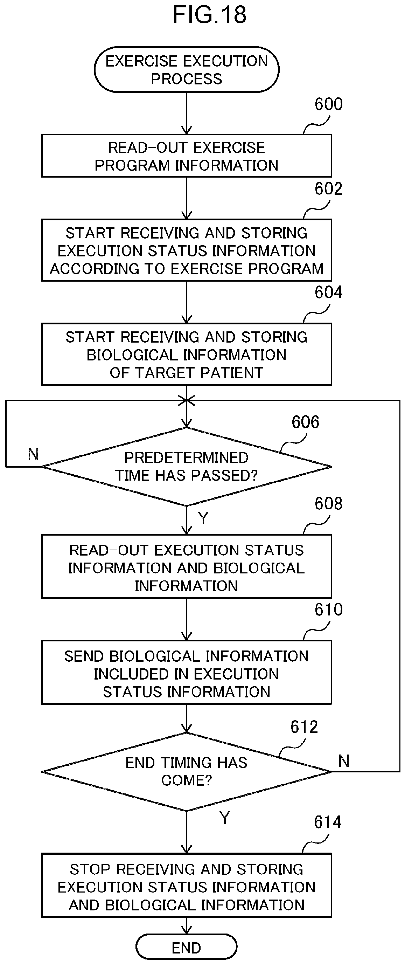

Next, the operation of the information processing device 30 when the exercise execution process is executed will be described with reference to Fig. 18. When the CPU 31 of the information processing device 30 executes the exercise execution program 33C, the exercise execution process illustrated in Fig. 18 is executed. The exercise execution process illustrated in Fig. 18 is executed in a case where an applicant who is permitted to enroll in the provided service (hereinafter, referred to as a "permitted person") executes an exercise in an enrolled sports club and when an instruction to execute the exercise execution process is input from a person in charge of the sports club via the input unit 34. Note that the permitted person puts the wearable terminal 50 possessed by the permitted person into an operating state and also puts the mobile terminal 40 possessed by the permitted person into an operating state. As a result, the information processing device 30 can continuously receive the biological information including the heart rate of the permitted person from the mobile terminal 40. Furthermore, in order to avoid complication herein, a case where the execution status information is constantly transmitted from the exercise apparatus group 60 provided in the corresponding sports club will be described.

In Step 600 of Fig. 18, the processing unit 31A reads the exercise program information corresponding to the permitted person from the storage unit 33. In Step 602, the processing unit 31 A starts receiving the execution status information from the exercise apparatus group 60 used in the exercise program indicated by the read exercise program information and storing the received execution status information in the storage unit 33. In Step 604, the processing unit 31A starts receiving the biological information from the mobile terminal 40 possessed by the permitted person and storing the received biological information in the storage unit 33.

In Step 606, the processing unit 31A waits until a predetermined time (in the present embodiment, 10 seconds) elapses, and in Step 608, the processing unit 31A reads, from the storage unit 33, the execution status information and the biological information stored from the predetermined time before going back from that time point to this time point.

In Step 610, the processing unit 31A includes the read biometric information in the read execution status information and transmits the read execution status information to the exercise support device 10 together with information indicating the patient ID of the permitted person. In Step 612, the processing unit 31A determines whether a predetermined end timing has come. If a negative determination is made, the process returns to Step 606, and if an affirmative determination is made, the process proceeds to Step 614. In the present embodiment, a timing at which the operating state of the wearable terminal 50 worn by the permitted person is stopped is applied as the end timing, but the present invention is not limited thereto. For example, a timing at which the person in charge inputs an instruction to stop transmitting the execution status information to the exercise support device 10 via the input unit 34 may be set as the end timing.

In Step 614, the processing unit 31A stops receiving the execution status information and the biometric information, started by the processing in Steps 602 and 604 respectively, and storing those information in the storage unit 33, and then ends the present exercise execution process.

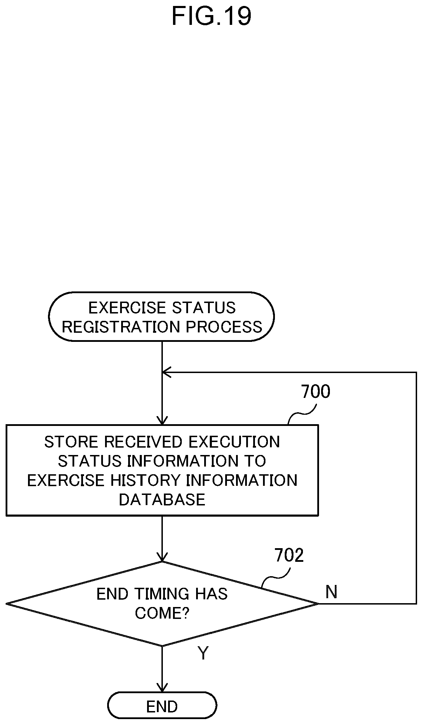

Next, an operation of the exercise support device 10 when the exercise status registration process according to the present embodiment is executed will be described with reference to Fig. 19. When the CPU 11 of the exercise support device 10 executes the exercise status registration program 13B, the exercise status registration process illustrated in Fig. 19 is executed. The exercise status registration process illustrated in Fig. 19 is executed when the execution status information is received by any sports club.

In Step 700 of Fig. 19, the execution unit 11B stores the received execution status information in the exercise history information database 13F as the exercise history corresponding to the patient ID received together with the execution status information. In Step 702, the execution unit 11B determines whether or not a predetermined end timing has come. If a negative determination is made, the process returns to Step 700. On the other hand, if an affirmative determination is made, the present exercise status registration process ends. In the present embodiment, as the end timing, a timing at which the execution status information from the sports club has not been received over the predetermined time is applied, but the present invention is not limited thereto. For example, with reference to the exercise program information provided to the person permitted corresponding to the received patient ID, the timing at which storing the execution status information in the storage unit 33 ends by a corresponding amount of exercise for one day indicated by the exercise program information may be set as the end timing.

As described above, according to the present embodiment, the exercise support device includes: the acquisition unit 11A that acquires, from a medical institution, confirmation result information indicating a confirmation result regarding the execution of an exercise of a patient at an exercise facility in which the exercise by an exercise program according to a disease of the patient who has visited the medical institution is performed; and the execution unit 11B that executes a process of transmitting, to both the exercise facility and the patient, permission or non-permission information of the execution of the exercise according to the exercise program of the patient at the exercise facility on the basis of the confirmation result information acquired by the acquisition unit 11A. Therefore, it is possible to reliably prevent a patient from performing an exercise at an exercise facility that has not been confirmed by a medical institution as to whether or not the patient can perform the exercise at the exercise facility.

In addition, according to the present embodiment, the exercise program information indicating the exercise program is provided to both the exercise facility and the patient. Therefore, convenience for the destination of the exercise program information can be improved.

Furthermore, according to the present embodiment, in addition to the exercise program information, the effect information indicating the effect of the exercise according to the exercise program information is provided to both the exercise facility and the patient. Therefore, it is possible to improve motivation for the patient to continue the exercise according to the exercise program.

In addition, according to the present embodiment, a charge corresponding to the use of the exercise facility by the patient is set. Therefore, it is possible to obtain compensation for the provision of the exercise program information.

In addition, according to the present embodiment, the amount of the charge includes at least a part of the cost for facility use received by the exercise facility from the patient. Therefore, more reasonable charging can be performed.

In addition, according to the present embodiment, payment of compensation for the provision of the exercise program information is instructed to the patient. Therefore, it is possible to save time and effort for newly creating a credit card or the like.

Furthermore, according to the present embodiment, the execution status information indicating the execution status of the exercise according to the exercise program of the patient is acquired, and the acquired execution status information is stored in a storage unit so as to be referable by a medical worker of the medical institution is further executed. Therefore, it is possible to cause the medical worker to easily grasp the execution status of the exercise by the patient.

Furthermore, according to the present embodiment, the execution status information is also acquired from the wearable terminal worn by the patient during the exercise. Therefore, the execution status information can be acquired more easily.

In addition, according to the present embodiment, the exercise program is a program by a combination of the aerobic exercise and the resistance exercise. Therefore, a more effective exercise program can be provided.

In addition, according to the present embodiment, intensity, duration, and type are defined as the aerobic exercise and the resistance exercise. Therefore, the aerobic exercise and the resistance exercise can be more easily and quantitatively defined.

In addition, according to the present embodiment, the exercise program is a program whose usefulness has been confirmed by a predetermined method. Therefore, it is possible to improve motivation for the patient to continue the exercise according to the exercise program.

In the present embodiment, as the predetermined method, the method for verifying usefulness by a parallel group comparison test of an exercise intervention group and a control group is applied. Therefore, the scientific usefulness of the exercise program itself can be verified.

Furthermore, according to the present embodiment, the disease is type 2 diabetes. Therefore, it is possible to reliably prevent a patient from performing an exercise at an exercise facility in which the advisability of performing an exercise for the type 2 diabetes disease has not been confirmed by a medical institution.

Note that, in the above embodiment, a case where patient information of a patient is transmitted to the information reception device 20 used by a doctor in charge by e-mail has been described, but the present invention is not limited thereto. For example, the patient information may be transmitted to the information reception device 20 via a dedicated line.

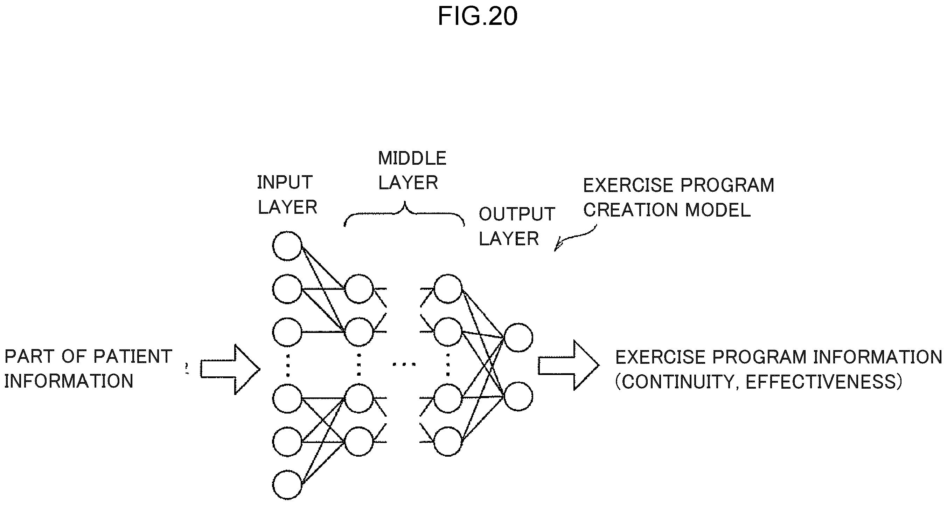

In addition, in the above embodiment, a case where the exercise program is prepared in advance on the assumption that the usefulness is confirmed for respective gender and age of the patient has been described, but the present invention is not limited thereto. For example, as an example, an exercise program creation model that is a machine learning model for creating exercise program information suitable for a patient illustrated in Fig. 20 may be created in advance, and the exercise program information may be created for each patient using the exercise program creation model.

Note that the exercise program creation model illustrated in Fig. 20 is a neural network including an input layer, a plurality of intermediate layers, and an output layer. The gender and the age of the target patient, which are a part of the patient information according to the above embodiment, are input to the input layer of the exercise program creation model. The output layer of the exercise program creation model outputs exercise program information suitable for the patient corresponding to the input information. The exercise program creation model is obtained in advance by causing a plurality of combinations of the gender and age of the patient obtained in the past and the exercise program information having high exercise continuity in the patient and having a high effect of improving the disease to be learned as teaching data.

In addition, in the above embodiment, the exemplary embodiment in a case where the present invention is applied when the patient is a patient with type 2 diabetes has been described, but the present invention is not limited thereto. For example, the present invention can also be applied to patients with other diseases whose symptoms may be improved by performing exercise by an exercise program, such as a case where the patient is a patient with metabolic syndrome.

In addition, in the above embodiment, the case where the confirmation by the medical institution is performed before the patient is enrolled at the exercise facility has been described, but the present invention is not limited thereto. For example, after enrollment of a patient at an exercise facility and during a period of performing exercise according to a provided exercise program at the exercise facility, confirmation may be performed at the medical institution with reference to the execution status information or the like registered in the exercise history information database 13F.

In the above embodiment, the case where the execution status information, which is information indicating the actual exercise status, is stored in the exercise history information database 13F has been described, but the present invention is not limited thereto. For example, in addition to the above-described execution status information, access record information indicating a record of access to a corresponding sports club may be stored in the exercise history information database 13F.

Furthermore, in the above-described embodiment, only the action at the time of executing the registration request process (see Fig. 7) has been described as the action of the mobile terminal 40, but the present invention is not limited thereto. For example, the mobile terminal 40 may be configured to play roles such as requesting for withdrawal from an enrolled exercise facility, acquiring information regarding access to the exercise facility by reading a two-dimensional code, a bar code, or the like, and receiving input of an exercise execution result.

Furthermore, in the above-described embodiment, a case where the non-permission information is transmitted to both the corresponding patient and the sports club in the registration confirmation processing (see Fig. 11) has been described, but the present invention is not limited thereto. For example, the non-permission information need not be transmitted to the sports club but may be transmitted only to the corresponding patient.

Furthermore, in the above embodiment, for example, the following various processors can be used as a hardware structure of a processing unit that executes each process of the acquisition unit 11A, the execution unit 11B, the provision unit 11C, the setting unit 11D, and the instruction unit 11E. As described above, the various processors include not only a CPU that is a general-purpose processor that functions as a processing unit by executing software (program), but also a dedicated electric circuit or the like that is a processor having a circuit configuration designed exclusively for executing specific processing such as a programmable logic device (PLD) that is a processor whose circuit configuration can be changed after manufacturing of a field-programmable gate array (FPGA) or an application specific integrated circuit (ASIC).

The processing unit may include one of these various processors, or may include a combination of two or more processors of the same type or different types (for example, a combination of a plurality of FPGAs or a combination of a CPU and an FPGA). In addition, the processing unit may be configured by one processor.

As an example in which the processing unit is configured by one processor, first, there is a mode in which one processor is configured by a combination of one or more CPUs and software as represented by a computer such as a client and a server, and this processor functions as a processing unit. Second, as represented by a system on chip (SoC) or the like, there is a form of using a processor that realizes a function of the entire system including a processing unit by one integrated circuit (IC) chip. As described above, the processing unit is configured using one or more of the above-described various processors as a hardware structure.

Furthermore, more specifically, an electric circuit (circuitry) obtained by combining circuit elements such as semiconductor elements can be used as a hardware structure of these various processors.

### (Supplementary notes)

With regard to the above embodiments, the following supplementary notes are further disclosed.

### (Supplementary note 1)

An exercise support device comprising:
a memory; and
at least one processor connected to the memory, wherein
the processor is configured to:
   acquire, from a medical institution, confirmation result information indicating a confirmation result regarding execution of an exercise of a patient at an exercise facility in which the exercise according to an exercise program based on a disease of the patient who has visited the medical institution is performed; and
   execute a process of transmitting, to at least one of the exercise facility or the patient, permission or non-permission information of the execution of the exercise according to the exercise program of the patient at the exercise facility on the basis of the acquired confirmation result information.

### (Supplementary note 2)

A non-transitory storage medium storing an executable program for causing a computer to execute an exercise support process, wherein
the exercise support process includes:
acquiring, from a medical institution, confirmation result information indicating a confirmation result regarding execution of an exercise performed by a patient who has visited the medical institution, at the exercise facility in which the exercise according to an exercise program based on a disease of the patient is performed; and
transmitting, to at least one of the exercise facility or the patient, permission or non-permission information of the execution of the exercise according to the exercise program of the patient at the exercise facility on the basis of the acquired confirmation result information.

The disclosure of Japanese Application No. 2019-199834 is incorporated herein by reference in its entirety.

All documents, patent applications, and technical standards described in this specification are incorporated herein by reference to the same extent as if each individual document, patent application, and technical standard were specifically and individually described to be incorporated by reference.

## Claims

1. An exercise support device comprising:
an acquisition unit that acquires, from a medical institution, confirmation result information indicating a confirmation result regarding execution of an exercise of a patient who has visited a medical institution at an exercise facility in which the exercise is performed by an exercise program according to a disease of the patient; and
an execution unit that executes a process of transmitting, to at least one of the exercise facility or the patient, permission or non-permission information of the execution of the exercise according to the exercise program of the patient at the exercise facility based on the confirmation result information acquired by the acquisition unit.

2. The exercise support device according to claim 1, further comprising:
a provision unit configured to provide exercise program information indicating the exercise program to at least one of the medical institution, the exercise facility, or the patient.

3. The exercise support device according to claim 2, wherein
the provision unit provides, in addition to the exercise program information, effect information indicating an effect of exercise according to the exercise program information to at least one of the medical institution, the exercise facility, or the patient.

4. The exercise support device according to claim 2 or 3, further comprising:
a setting unit that sets a charge corresponding to use of the exercise facility by the patient in a case in which a destination to which the exercise program information is provided by the provision unit is the exercise facility.

5. The exercise support device according to claim 4, wherein
an amount of the charge includes at least a part of a cost for facility use received by the exercise facility from the patient.

6. The exercise support device according to claim 2 or 3, further comprising:
an instruction unit that instructs payment of compensation for provision of the exercise program information to the patient in a case in which a destination to which the exercise program information is provided by the provision unit is the patient.

7. The exercise support device according to any one of claims 1 to 6, wherein:
the acquisition unit further acquires execution status information indicating an execution status of exercise according to the exercise program of the patient, and
the execution unit further executes a process of storing the execution status information acquired by the acquisition unit in a storage unit so that a medical worker of the medical institution can refer to the execution status information.

8. The exercise support device according to claim 7, wherein
the acquisition unit acquires the execution status information from a wearable terminal worn by the patient during the exercise.

9. The exercise support device according to any one of claims 1 to 8, wherein
the exercise program is a program by a combination of aerobic exercise and resistance exercise.

10. The exercise support device according to claim 9, wherein
the aerobic exercise and the resistance exercise are defined in at least one of frequency, intensity, duration, or type.

11. The exercise support device according to any one of claims 1 to 10, wherein
the exercise program is a program whose usefulness has been confirmed by a predetermined method.

12. The exercise support device according to claim 11, wherein
the predetermined method is a method of verifying the usefulness by a parallel group comparison test of an exercise intervention group and a control group.

13. The exercise support device according to any one of claims 1 to 12, wherein
the disease is at least one of metabolic syndrome or type 2 diabetes.

14. An exercise support system comprising:
the exercise support device according to any one of claims 1 to 13;
an information reception device provided in the medical institution and including a reception unit that receives the confirmation result information and a transmission unit that transmits the confirmation result information to the exercise support device; and
an information processing device provided at the exercise facility, the information processing device including a processing unit that performs processing according to a process of transmitting permission or non-permission information for execution of an exercise according to the exercise program at the exercise facility, by the execution unit in the exercise support device.

15. An exercise support method comprising:
acquiring, from a medical institution, confirmation result information indicating a confirmation result regarding execution of an exercise of a patient who has visited the medical institution at an exercise facility in which the exercise, according to an exercise program based on a disease of the patient, is performed; and
executing a process of transmitting, to at least one of the exercise facility or the patient, permission or non-permission information of execution of the exercise according to the exercise program of the patient at the exercise facility based on the acquired confirmation result information.

16. A program for causing a computer to execute a process of
acquiring, from a medical institution, confirmation result information indicating a confirmation result regarding execution of an exercise of a patient who has visited the medical institution at an exercise facility in which the exercise, according to an exercise program based on a disease of the patient, is performed; and
executing a process of transmitting, to at least one of the exercise facility or the patient, permission or non-permission information of execution of the exercise according to the exercise program of the patient at the exercise facility based on the acquired confirmation result information.
